# FASCICULE DE BREVET EUROPEEN

(11) **EP 1 567 485 B1**
(45) Date de publication et mention de la délivrance du brevet: **19.01.2011**
(21) Numéro de dépôt: 03782554.4
(22) Date de dépôt: 13.11.2003
(51) Int. Cl.: C07C 311/03, C02F 1/62

(54) **SELS DE PERFLUOROALKYLE SULFONAMIDES COMME AGENTS COMPLEXANTS**
SALZEN DER PERFLUOROALKYLSULFONAMIDEN ALS KOMPLEXBILDNER
PERFLUOROALKYLE SULPHONAMIDE SALTS AND THEIR USE AS COMPLEXING AGENTS

(30) Priorité: 15.11.2002 FR 0214334
(43) Date de publication de la demande: 31.08.2005
(73) Titulaire: CENTRE NATIONAL DE LA RECHERCHE SCIENTIFIQUE (CNRS), 75016 Paris (FR); Université de Montpellier II, 34095 Montpellier Cedex 5 (FR)
(72) Inventeur: BLANCOU, Hubert, F-34480 PUISSALICON (FR); GUILLEN, Franck, F-34310 MONTADY (FR)
(74) Mandataire: Colombet, Alain André
(86) Numéro de dépôt international: PCT/FR2003/003368
(87) Numéro de publication internationale: WO 2004/046093

(56) Documents cités:
- EP-A- 0 331 576
- US-A- 4 370 254

## Description

La présente invention concerne des composés de formule R_{f}SO₂NH⁻Y⁺ où R_{f} représente un groupement perfluoroalkyle en C₇-C₉ et Y⁺ un cation, leur procédé de préparation et leur utilisation à titre d'agents complexants ainsi qu'un procédé de récupération de cations polyvalents mettant en oeuvre ces agents complexants.

Différentes méthodes permettant de réaliser une séparation cationique sont connues à ce jour.

Ainsi, la séparation cationique peut être réalisée par voie « physique » en utilisant des membranes ou en procédant à une électrodialyse, une électrodialyse avec membrane, une électrodialyse à membrane bipolaire, une dialyse ionique, une osmose inverse, une pervaporation, une perméation gazeuse. Cependant, ces procédés s'appliquent à des cations particuliers et s'avèrent généralement onéreux.

La séparation cationique peut également être effectuée par «voie chimique », par précipitation des cations sous forme de sels insolubles. Cette méthode, d'une grande simplicité de mise en oeuvre et d'une spécificité relativement élevée, présente, cependant, des performances limitées par les produits de solubilité des précipités qui en résultent. D'autres méthodes de séparation cationique par voie chimique utilisent des complexants ou chélatants moléculaires ou supportés sur résine qui permettent la récupération de cations par extraction avec des solvants adaptés. A titre illustratif de telles molécules complexantes, on peut notamment citer les éthers couronnes, les cryptants, les podants, les calixarènes. Pour plus d'informations concernant ces composés, on pourra notamment se référer aux ouvrages suivants : B. Dietrich, P. Viout, J-M. Lehn, « Aspects de la chimie des composés macrocycliques », Inter Edition du CNRS, Paris, Meudon, 1991, 90-92*. ;* P.S. Vankar, V. Tiwari, (FEAT), Indian Institute of Technology, Journal of Analytical Science, 2001 ref 2001-BA006*.* Ces complexants spécifiques d'un cation ou d'un type de cation donné sont cependant d'un coût très élevé.

La séparation cationique peut également être réalisée par « voie biologique » comme il l'a été décrit notamment dans la publication *T. Sakaguchi, M. Iwatsuki, AJINOMOTO. C. O*, *INC-JP,* FR 2639652, 1989. Ainsi, les cations peuvent être consommés sélectivement par des bactéries, la récupération des cations s'effectuant après séparation des bactéries du milieu et la lyse de celles-ci. Cependant, ce type de procédé est coûteux, relativement difficile à mettre en oeuvre et requiert des compétences techniques spécifiques relevant de la biochimie.

De façon inattendue, il a été démontré que certains composés de formule R_{f}SO₂NH-Y⁺ où R_{f} représente un groupement perfluoroalkyle en C₇-C₉ sont solubles et stables en phase aqueuse neutre et ont la particularité de complexer les cations de type Mⁿ⁺ (où n est un entier >1 représentant la valence du cation) en solution aqueuse neutre sous forme d'un complexe insoluble, celui-ci résultant de l'agrégation de plusieurs particules de complexes insolubles centrées sur les cations Mⁿ⁺.

Ceci permet, avantageusement, la récupération physique des cations après précipitation du complexe insoluble, filtration et traitement acide du solide récupéré, ainsi que leur éventuelle valorisation.

De façon avantageuse, ces composés de formule R_{f}SO₂NH⁻Y⁺ permettent la récupération d'une grande variété de cations de valence > 1 en solution aqueuse, ceci grâce à une complexation souple, par opposition aux complexations rigides réalisées notamment avec des structures rigides de type éther couronne ou calixarène. Ils peuvent être ainsi assimilés à des « éponges à cations polyvalents ».

De plus, les composés de formule R_{f}SO₂NH⁻Y⁺ forment des complexes particulièrement hydrophobes en présence de cations polyvalents, grâce à l'apport original de l'hydrophobie des groupements fluorés, assurant ainsi une bonne précipitation.

Avantageusement, les composés de formule R_{f}SO₂NH⁻Y⁺ se caractérisent par une bonne densité (généralement voisine de 2) et par là, du complexe, pouvant permettre une centrifugation.

De façon particulièrement avantageuse, on observe une bonne agrégation des particules de complexe, résultant d'interactions par ségrégation de phase et forces faibles des groupements fluorés, permettant la filtration.

Enfin, les composés de formule R_{f}SO₂NH⁻Y⁺ ont un coût relativement faible par rapport aux complexants classiques, en général 120 € par kg pour l'agent complexant qui est de plus recyclable.

La présente invention a donc pour but de proposer de nouveaux agents complexants très performants.

Ces composés sont notamment utiles pour le traitement des eaux et des effluents, et particulièrement pour l'extraction de métaux lourds comme par exemple le plomb, le cadmium, le chrome.

L'invention a également pour but de proposer un procédé de séparation cationique performant, facile à mettre en oeuvre, peu coûteux, et généralisable à la plupart des cations polyvalents.

Ces buts et d'autres peuvent être atteints par la présente invention qui concerne un composé de formule R_{f}SO₂NH⁻⁺Y, dans laquelle R_{f} représente un groupement perfluoroalkyle en C₇-C₉.

En effet, les groupements perfluoroalkyles comprenant moins de 7 atomes de carbone sont solubles en milieu aqueux, mais ne forment pas de complexe insoluble avec un cation polyvalent, Mⁿ⁺. Les groupements perfluoroalkyles comprenant plus de 9 atomes de carbone sont en revanche insolubles dans l'eau.

Bien entendu, tout groupement perfluoré ou perhalogéné en α de la fonction sulfamide susceptible d'induire de telles propriétés, à savoir solubilité et stabilité en solution aqueuse neutre d'une part, complexation d'un cation polyvalent sous forme d'un complexe insoluble d'autre part, est également compris dans l'invention.

Le terme "perfluoroalkyle en C₇-C₉" au sens de la présente description désigne un groupement répondant à la formule CₙF₂ₙ₊₁ avec n = 7, 8 ou 9, pouvant être linéaire ou ramifié, ou bien cyclique de formule CₙF₂ₙ₋₁ avec n = 7, 8 ou 9.

Plus préférentiellement, R_{f} représente un groupement perfluoroalkyle en C₇-C₉ linéaire.

On préfère tout particulièrement un groupement R_{f} comprenant 8 atomes de carbones.

Le cation Y⁺ est un cation monovalent, de préférence inorganique. Il est particulièrement choisi parmi les cations de métaux alcalins tels que Na⁺, Li⁺, K⁺, et l'ammonium NH₄⁺.

Les composés selon l'invention peuvent être avantageusement préparés à partir de composés sulfamides de formule R_{f}SO₂NH₂, où R_{f} est tel que défini précédemment.

Ce procédé comprend les étapes de :
a1) mise en contact d'un composé R_{f}SO₂NH₂ avec une base BY dans un solvant ;
b1) récupération du composé R_{f}SO₂NH⁻⁺Y.

Le solvant mis en oeuvre dans ce procédé est choisi parmi les solvants polaires, et préférentiellement parmi les alcools tels que le méthanol ou l'éthanol, les éthers, les esters aliphatiques.

De préférence, la base BY est une base dont le pKa est supérieur à 6, plus préférentiellement supérieur à 10. A titre illustratif, on peut citer notamment les bases CH₃OY, CO₃Y₂, YOH où Y peut représenter un cation de métal alcalin ou un ammonium.

Les composés sulfamides de formule R_{f}SO₂NH₂ où R_{f} représente une chaîne perfluoro alkyle en C₇-C₉ peuvent être préparés selon un procédé de synthèse analogue à celui décrit dans la publication Li-Quing Hu and Darryl D. DesMarteau, Inorg. Chem. (1993) 32, 5007-5010.

L'invention a également pour objet, selon un autre aspect, un procédé de séparation de cations polyvalents Mⁿ⁺ (n>1) comprenant les étapes de :
(i) mise en contact d'une solution aqueuse de pH approprié contenant lesdits cations avec une quantité suffisante de composé R_{f}SO₂NH⁻⁺Y ;
(ii) récupération du complexe (R_{f}SO₂NH⁻)ₙM^{n+,} formé en solution aqueuse ;
(iii) dissociation du complexe par traitement avec un acide HX ;
(iv) séparation du composé R_{f}SO₂NH₂ et récupération du cation n(X)Mⁿ⁺.

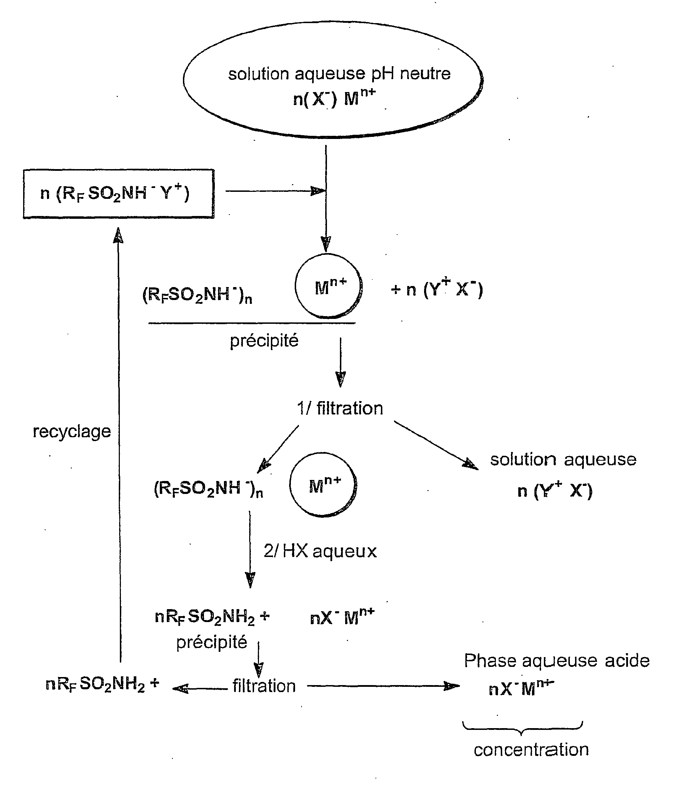

La figure jointe en annexe représente de façon schématique et illustrative le principe général de ce procédé.

Un pH approprié, au sens de la présente description, signifie un pH compris entre le pKa du couple acide-base conjugué R_{f}SO_{2N}H₂ / R_{f}SO₂NH⁻ et le pKb du couple Mⁿ⁺/ MM(OH)ₙ, de préférence entre 6,5 et 7,5, mieux encore entre 6,8 et 7,2.

En effet, en milieu acide, soit à un pH<pKa, le composé R_{f}SO₂NH⁻Y⁺ est instable par rapport à sa forme acide conjuguée R_{f}SO₂NH₂.

En milieu basique, soit à un pH >pKb, le cation Mⁿ⁺ est instable comparativement à sa forme hydroxylée M(OH)ₙ: Mⁿ⁺ + OH⁻ ⇄ M(OH)ₙ.

Une « quantité suffisante de composé R_{f}SO₂NH⁻⁺Y » désigne une quantité permettant de complexer la quantité souhaitée d'ions Mⁿ⁺, de préférence au moins égale à n équivalents molaires de cations polyvalents Mⁿ⁺, n étant un entier >2, préférentiellement en faible excès, mieux encore proche de 1,5 n équivalents. Selon un mode de réalisation privilégié, la solution aqueuse contenant lesdits cations, Mⁿ⁺ n'est pas trop diluée, la complexation des ions Mⁿ⁺ par les composés R_{f}SO₂NH⁻Y⁺ étant d'autant plus efficace que les ions Mⁿ⁺ sont concentrés. Avantageusement, la dilution de la solution est non inférieure à 25 ppm, mieux encore à 50 ppm.

Bien entendu, il est des compétences de l'homme du métier d'ajuster la quantité de composé R_{f}SO₂NH⁻Y⁺ en fonction de la nature du cation Mⁿ⁺ à complexer et de la dilution de la solution contenant lesdits cations, de manière à obtenir des conditions de complexation optimales.

Avantageusement, le complexe insoluble est récupéré dans l'étape (ii) par filtration ou centrifugation.

De préférence, la dissociation du complexe insoluble de l'étape (iii) est réalisée par traitement avec un acide HX possédant un pKa < 3, plus préférentiellement un pKa < 2.

L'acide mis en oeuvre est choisi notamment parmi l'acide chlorhydrique et l'acide sulfurique.

Le composé R_{f}SO₂NH₂ obtenu après traitement acide à l'issue de l'étape (iii) étant insoluble dans l'eau, précipite et peut être avantageusement séparé de la solution aqueuse contenant les cations Mⁿ⁺ par filtration, dans l'étape (iv). Les cations sont récupérés dans le filtrat sous la forme n(X⁻)Mⁿ⁺ et sont éventuellement concentrés.

Avantageusement, les ions Mⁿ⁺ sont récupérés avec d'excellents rendements généralement supérieurs à 99%, voire 99,9% dans des conditions optimisées.

Avantageusement, le complexant R_{f}SO₂NH⁻⁺Y peut être ensuite régénéré à partir de R_{f}SO₂NH₂ ainsi isolé, selon une étape (v) subséquente, dans les conditions du procédé de préparation du composé R_{f}SO₂NH⁻⁺Y précité.

De façon particulièrement avantageuse, le complexant R_{f}SO₂NH⁻⁺Y peut être réutilisé.

A titre d'exemples de cations polyvalents susceptibles d'être récupérés selon ce procédé, ont peut notamment citer les métaux lourds comme le plomb, le cadmium, le chrome, les cations provenant de déchets électriques ou électroniques comme le cuivre ou les terres rares, des ions métalliques à activité catalytiques comme les métaux de transition tels que le cobalt, le rhodium, le palladium.

Selon un autre aspect, l'invention a pour objet les composés de formule (R_{f}SO₂NH⁻)ₙ Mⁿ⁺, où M⁺, n et R_{f} ont les définitions précitées, et où

M représente un métal choisi parmi le plomb, le cadmium, le chrome, le cuivre, les terres rares et les métaux de transition.

L'invention vise également l'utilisation des composés de formule R_{f}SO₂NH⁻⁺Y à titre d'agent complexant de cations polyvalents Mⁿ⁺ avec n>1.

Entre également dans l'esprit de l'invention l'utilisation de mélanges de composés de formule R_{f}SO₂NH⁻Y⁺.

Selon une variante de l'invention, les composés de formule R_{f}SO₂NH⁻Y⁺ peuvent être utilisés sous forme immobilisée sur un support solide.

Ces composés sont notamment utiles pour la récupération de cations polyvalents dans les eaux naturelles, minérales ou thermales, dans les effluents industriels et ménagers, dans les solutions (neutralisées) résultant de traitement des déchets (récupération du cuivre ou des terres rares provenant de déchets électriques ou électroniques), épuration des eaux et effluents (extraction des métaux lourds comme le Pb, Cd, Cr ...).

### EXEMPLES

### Exemple 1 : Synthèse des agents complexants :

### Synthèse de sulfamide C₈R₁₇SO₂NH₂.

0,1 mole de C₈F₁₇SO₂F (50,1 g) en solution dans un solvant anhydre (acétate d'éthyle, chloroforme, éther....) est soumise à une température de 15-20°C à un courant d'ammoniac préalablement séché sur potasse. L'avancement réactionnel est suivi en RMN ¹⁹F par intégration des signaux caractéristiques des CF₂ fonctionnels ; CF₂SO₂F et CF₂SO₂NH₂.

A la fin de la réaction, le solvant organique est évaporé sous pression réduite, les cristaux obtenus en solution dans 50 mL d'acétate d'éthyle sont lavés par 50 mL d'HCl 10 %. Le solvant de la phase organique qui contient le sulfamide est ensuite évaporé. On récupère 47 g de C₈F₁₇SO₂NH₂ (rendement : 95 %).

### Synthèse du C₈ H₁₇ SO₂ NH⁻Y⁺, (M = Na, Li, K, NH₄...)

0,1 mole de C₈ H₁₇ SO₂ NH₂ en solution dans 20 mL de méthanol ou d'éthanol anhydre est mise en contact à 25°C avec 0,1 mole d'une des bases suivantes (CH₃O⁻⁺Na, CO₃⁻ (Na⁺,Li⁺, K⁺, NH₄⁺...), (Na⁺,Li⁺, K⁺)⁻OH, NaNH₂).

Le sel C₈ H₁₇ SO₂ NH⁻Y⁺ de la base correspondante précipite dans le milieu et est récupéré par filtration (rendement quantitatif).

### APPLICATION

### Exemple 2 : Extraction de différents cations polyvalents et régénération du complexant

Les solutions contenant différentes concentrations de sels, indiquées dans le tableau 1, dissous dans de l'eau distillée sont préparées.

A chacune de ces solutions, on ajoute différentes concentrations de l'agent complexant (C₈F₁₇SO₂NH⁻⁺Na) qui au départ est soluble dans l'eau. Après agitation (environ une minute), on observe un précipité résultant de la complexation du cation présent dans la solution.

La solution est ensuite filtrée à l'aide d'un filtre nylon de 0,4 micron, le filtrat est ensuite analysé par ICPMS (Spectromètre de masse quadripolaire à source plasma), les résultats obtenus sont résumés dans le tableau ci-dessous. Le rétentat (complexe composé de n molécules d'agent complexant et du métal Mⁿ⁺) est repris par une solution aqueuse d'HCl, ce qui permet le relargage du cation sous forme de chlorure et la précipitation de l'agent sous forme C₈F₁₇SO₂NH₂. Après filtration l'agent complexant peut être réutilisé.

**Tableau 1 de résultats : concentrations en PPM des différents cations avant et après traitement par le complexant.**

| Ref | Cation Mⁿ⁺ | Forme du cation | *R_{f}SO₂NH⁻Y⁺*/ *Cation Mⁿ⁺* | Concentration du cation (PPM) | Concentration du cation après traitement PPM |
|---|---|---|---|---|---|
| A | UO₂++ | Acétate dihydrate | 3,8 | 560 | 22 |
| B | Cu++ | Acétate dihydrate | 2,16 | 209 | 0,175 |
| C | Cu++ | | 2,5 | 195 | 0,043 |
| D | Co++ | Bromure hexahydrate | 2,6 | 1160 | 7,3 |
| E | Pb++ | Acétate trihydrate | 2,3 | 220 | 28 |
| F | Cd++ | Acétate dihydrate | 2,2 | 213 | 18 |
| G | Sr++ | Chlorure hexahydrate | 2,17 | 200 | 28 |

Ce procédé permet de récupérer de façon efficace, les cations étudiés présents en solution aqueuse de pH compris entre 6,8 et 7,2.

Avantageusement, cette méthode permet de récupérer de 80% à 99% des cations en solution.

### Exemple 3 : Optimisation des conditions d'extraction du plomb

Une étude visant à optimiser la quantité de plomb récupérée selon le procédé de l'invention a été réalisée.

**Tableau 2 de résultats : concentrations en PPM des cations avant et après traitement par le complexant en fonction du rapport R = concentration en complexant/concentration en cation.**

| Ref | Cation Mⁿ⁺ | Forme du cation | *R_{f}SO₂NH*⁻*Y*⁺/ *Cation Pb2*⁺ | Concentration du cation PPM | Concentration du cation après traitement PPM |
|---|---|---|---|---|---|
| 1 | Pb++ | Acétate trihydrate | 1 | 500 | 481 |
| 2 | Pb++ | Acétate trihydrate | 2 | 500 | 187 |
| 4 | Pb++ | Acétate trihydrate | 3 | 500 | 0,54 |
| 5 | Pb++ | Acétate trihydrate | 4 | 500 | 2,2 |

Ces résultats montrent que le rendement de récupération varie en fonction du rapport de la concentration molaire de l'agent complexant R_{f}SO₂NH⁻Y⁺ avec celle du cation Pb²⁺. Ainsi, une quantité sensiblement supérieure à la stoechiométrie, par exemple 1,5 n (réf. 4) ou 2 n (réf. 5), permet la récupération de plus de 99,5% des ions Pb²⁺ en solution, 1,5 n représentant la quantité de complexant permettant d'atteindre un rendement de récupération optimal de 99,9%.

**Tableau 3 de résultats : concentrations en PPM avant et après traitement par le complexant en fonction de la concentration en Pb de départ.**

| Ref | Cation Mⁿ⁺ | Forme du cation | *R_{f}SO₂ NH*⁻*Y*⁺ / *Cafion Pb*^{*2*+} | Concentration du cation PPM | Concentration du cation après traitement PPM |
|---|---|---|---|---|---|
| 4 | Pb++ | Acétate trihydrate | 3 | 500 | 0,54 |
| 6 | Pb++ | Acétate trihydrate | 3 | 300 | 0,19 |
| 7 | Pb++ | Acétate trihydrate | 3 | 200 | 0,3 |
| 8 | Pb++ | Acétate trihydrate | 3 | 100 | 0,6 |
| 9 | Pb++ | Acétate trihydrate | 3 | 75 | 0,28 |
| 10 | Pb++ | Acétate trihydrate | 3 | 50 | 0,56 |
| 11 | Pb++ | Acétate trihydrate | 3 | 25 | 3,1 |

Les résultats reportés dans le tableau 3 montrent que, pour un rapport [R_{f}SO₂NH⁻Y⁺ / Pb²⁺] fixé, le rendement de récupération des ions Pb²⁺ diminue sensiblement avec la dilution du milieu entre 99,9% et 98,9% pour une concentration variant entre 500 et 50 ppm. Cet effet devient notable pour les concentrations inférieures à 50 ppm, même si à 25 ppm le rendement de 87,5% reste très satisfaisant.

La récupération des ions Pb²⁺ selon le procédé de l'invention est donc d'autant plus efficace que le milieu est concentré.

## Revendications

1. Utilisation d'un composé de formule R_{f}SO₂NH⁻⁺Y, dans laquelle R_{f} représente un groupement perfluoroalkyle en C₇-C₉, et Y⁺ représente un cation inorganique, à titre d'agent complexant de cations polyvalents Mⁿ⁺ avec n>1.

2. Utilisation selon la revendication 1, dans laquelle R_{f} représente un groupe linéaire.

3. Utilisation selon l'une quelconque des revendications 1 ou 2, dans laquelle le groupement R_{f} comprend 8 atomes de carbone.

4. Utilisation selon l'une quelconque des revendications 1 à 3, dans laquelle Y⁺ représente un cation de métal alcalin ou un ammonium.

5. Utilisation selon l'une quelconque des revendications 1 à 4, pour le traitement des eaux et des effluents.

6. Utilisation selon l'une quelconque des revendications 1 à 5, **caractérisée en ce que** les cations polyvalents Mⁿ⁺ sont choisis parmi les métaux lourds.

7. Utilisation selon la revendication 6, dans laquelle les métaux lourds sont le plomb, le cadmium ou le chrome.

8. Procédé de récupération de cations polyvalents Mⁿ⁺ (n>1) comprenant les étapes de :
i) mise en contact d'une solution aqueuse de pH approprié contenant lesdits cations avec une quantité suffisante de composé R_{f}SO₂NH⁻⁺Y tel que défini dans les revendications 1 à 4 ;
ii) récupération du complexe (R_{f}SO₂NH⁻)ₙMⁿ⁺ formé en solution aqueuse ;
iii) dissociation du complexe par traitement avec un acide HX ;
iv) séparation du composé R_{f}SO₂NH₂ et récupération du cation n(X⁻)Mⁿ⁺.

9. Procédé selon la revendication 8, dans lequel l'étape ii) est une filtration ou une centrifugation.

10. Procédé selon l'une quelconque des revendications 8 ou 9, dans lequel l'acide HX possède un pKa < 3.

11. Procédé selon la revendication 10, dans lequel l'acide HX est l'acide chlorhydrique.

12. Procédé selon l'une quelconque des revendications 8 à 11, dans lequel l'étape iv) consiste en une séparation du composé R_{f}SO₂NH₂ par filtration ou extraction.

13. Procédé selon l'une quelconque des revendications 8 à 12, comprenant une étape v) subséquente de régénération du composé R_{f}SO₂NH⁻⁺Y à partir de R_{f}SO₂NH₂ selon le procédé comprenant :
a1) la mise en contact d'un composé R_{f}SO₂NH₂ avec une base BY dans un solvant ;
a2) la récupération du composé R_{f}SO₂NH₂.

14. Procédé selon la revendication 13, dans lequel le solvant est polaire.

15. Procédé selon la revendication 14, dans lequel le solvant est choisi parmi les alcools, les éthers, les esters aliphatiques.

16. Procédé selon l'une quelconque des revendications 13 à 15, dans lequel la base BY possède un pKa supérieur à 6.

17. Procédé selon la revendication 16, dans lequel la base BY est choisie parmi CH₃OY, CO₃Y₂, YOH, NH₂Y, où Y⁺ représente un cation de métal alcalin ou un ammonium.

18. Composé de formule (R_{f}SO₂NH⁻)ₙ, Mⁿ⁺,où M⁺ représente un cation de valence n, n étant un entier supérieur à 1, et R_{f} est tel que défini dans les revendications 1 à 4, dans lequel M représente un métal choisi parmi le plomb, le cadmium, le chrome, le cuivre, les terres rares et les métaux de transition.

## Claims

1. Use of a compound of formula R_{f}SO₂NH⁻Y⁺, wherein R_{f} represents a C₇-C₉perfluoroalkyl group and Y⁺ represents an inorganic cation, as a complexing agent for polyvalent cations Mⁿ⁺ where n > 1.

2. Use according to claim 1, wherein R_{f} represents a linear group.

3. Use according to any one of claims 1 or 2, wherein the R_{f} group comprises 8 carbon atoms.

4. Use according to any one of claims 1 to 3, wherein Y⁺ represents an alkali metal cation or ammonium.

5. Use according to any one of claims 1 to 4, in the treatment of water and effluents.

6. Use according to any one of claims 1 to 5, **characterised in that** the polyvalent cations Mⁿ⁺ are selected from the heavy metals.

7. Use according to claim 6, wherein the heavy metals are lead, cadmium or chromium.

8. Process for recovery of polyvalent cations Mⁿ⁺ (n > 1), comprising the steps:
i) bringing an aqueous solution of suitable pH containing said cations into contact with a sufficient amount of compound R_{f}SO₂NH⁻Y⁺ as defined in claims 1 to 4;
ii) recovery of the (R_{f}SO₂NH⁻)ₙMⁿ⁺ complex formed in aqueous solution;
iii) dissociation of the complex by treatment with an acid HX;
iv) separation of the compound R_{f}SO₂NH₂ and recovery of the cation N(X⁻)Mⁿ⁺.

9. Process according to claim 8, wherein step ii) is a filtration or a centrifugation.

10. Process according to any one of claims 8 or 9, wherein the acid HX has a pKa of < 3.

11. Process according to claim 10, wherein the acid HX is hydrochloric acid.

12. Process according to any one of claims 8 to 11, wherein step iv) consists of a separation of the compound R_{f}SO₂NH₂ by filtration or extraction.

13. Process according to any one of claims 8 to 12, comprising a subsequent step v) of regeneration of the compound R_{f}SO₂NH⁻Y⁺ starting from R_{f}SO₂NH₂, in accordance with the process comprising:
a1) bringing a compound R_{f}SO₂NH₂ into contact with a base BY in a solvent;
a2) recovery of the compound R_{f}SO₂NH⁻Y⁺.

14. Process according to claim 13, wherein the solvent is polar.

15. Process according to claim 14, wherein the solvent is selected from alcohols, ethers and aliphatic esters.

16. Process according to any one of claims 13 to 15, wherein the base BY has a pKa of more than 6.

17. Process according to claim 16, wherein the base BY is selected from CH₃OY, CO₃Y₂, YOH and NH₂Y, where Y⁺ represents an alkali metal cation or ammonium.

18. Compound of formula (R_{f}SO₂NH⁻)ₙMⁿ⁺, where Mⁿ⁺ represents a cation of valency n, n being an integer greater than 1, and R_{f} is as defined in claims 1 to 4, wherein M represents a metal selected from lead, cadmium, chromium, copper, the rare earths and the transition metals.

## Patentansprüche

1. Verwendung einer Verbindung der Formel R_{f}SO₂NH⁻Y⁺, wobei R_{f} für eine C₇-C₉-Perfluoralkylgruppe steht und Y⁺ für ein anorganisches Kation steht, als Komplexbildner mit mehrwertigen Kationen Mⁿ⁺, mit n > 1.

2. Verwendung nach Anspruch 1, wobei R_{f} für eine lineare Gruppe steht.

3. Verwendung nach einem der Ansprüche 1 oder 2, wobei die Gruppe R_{f} 8 Kohlenstoffatome umfasst.

4. Verwendung nach einem der Ansprüche 1 bis 3, wobei Y⁺ für ein Alkalimetallkation oder ein Ammonium steht.

5. Verwendung nach einem der Ansprüche 1 bis 4 zur Aufbereitung von Wasser und Abwasser.

6. Verwendung nach einem der Ansprüche 1 bis 5, **dadurch gekennzeichnet, dass** die mehrwertigen Kationen Mⁿ⁺ aus Schwermetallen ausgewählt sind.

7. Verwendung nach Anspruch 6, wobei es sich bei den Schwermetallen um Blei, Cadmium oder Chrom handelt.

8. Verfahren zur Gewinnung von mehrwertigen Kationen Mⁿ⁺ (n > 1), das die folgenden Schritte umfasst:
i) Inkontaktbringen einer wässrigen Lösung mit geeignetem pH-Wert, die die Kationen enthält, mit einer ausreichenden Menge der wie in den Ansprüchen 1 bis 4 definierten Verbindung R_{f}SO₂NH⁻Y⁺;
ii) Gewinnen des in der wässrigen Lösung gebildeten Komplexes (R_{f}SO₂NH⁻)ₙMⁿ⁺;
iii) Dissoziieren des Komplexes durch Behandlung mit einer HX-Säure;
iv) Trennen der Verbindung R_{f}SO₂NH₂ und Gewinnen des Kations n(X⁻)Mⁿ⁺_{.}

9. Verfahren nach Anspruch 8, wobei es sich bei dem Schritt ii) um eine Filtration oder Zentrifugation handelt.

10. Verfahren nach einem der Ansprüche 8 oder 9, wobei die HX-Säure einen pKa-Wert von < 3 hat.

11. Verfahren nach Anspruch 10, wobei es sich bei der HX-Säure um Salzsäure handelt.

12. Verfahren nach einem der Ansprüche 8 bis 11, wobei der Schritt iv) aus einer Trennung der Verbindung R_{f}SO₂NH₂ durch Filtration oder Extraktion besteht.

13. Verfahren nach einem der Ansprüche 8 bis 12, das einen Schritt v) nach der Regenerierung der Verbindung R_{f}SO₂NH⁻Y⁺ aus R_{f}SO₂NH₂ gemäß dem Verfahren umfasst, der Folgendes umfasst :
a1) Inkontaktbringen einer Verbindung R_{f}SO₂NH₂ mit einer BY-Base in einem Lösungsmittel;
a2) Gewinnen der Verbindung R_{f}SO₂NH⁻Y⁺.

14. Verfahren nach Anspruch 13, wobei das Lösungsmittel polar ist.

15. Verfahren nach Anspruch 14, wobei das Lösungsmittel aus Alkoholen, Ethern, aliphatischen Estern ausgewählt ist.

16. Verfahren nach einem der Ansprüche 13 bis 15, wobei die BY-Base einen pKa-Wert von mehr als 6 hat.

17. Verfahren nach Anspruch 16, wobei die BY-Base aus CH₃OY, CO₃Y₂, YOH, NH₂Y ausgewählt ist, worin Y⁺ für ein Alkalimetallkation oder ein Ammonium steht.

18. Verbindung der Formel (R_{f}SO₂NH⁻)ₙMⁿ⁺, worin Mⁿ⁺ für ein Kation mit der Wertigkeit n steht, n eine ganze Zahl von mehr als 1 ist und R_{f} wie in den Ansprüchen 1 bis 4 definiert ist, wobei M für ein Metall steht, das aus Blei, Cadmium, Chrom, Kupfer, Seltenerdmetallen und Übergangsmetallen ausgewählt ist.
